# EUROPÄISCHE PATENTSCHRIFT

(11) **EP 2 385 101 B1**
(45) Veröffentlichungstag und Bekanntmachung des Hinweises auf die Patenterteilung: **16.09.2020**
(21) Anmeldenummer: 11003535.9
(22) Anmeldetag: 29.04.2011
(51) Int. Cl.: C12M 3/00

(54) **Inkubator mit mehreren Ein- und Ausgabestationen und Transportvorrichtungen**
Incubator with multiple insertion and dispensing stations provided with means for transporting
Incubateur dotée de plusieurs stations d'entrée et muni de moyen de transport

(30) Priorität: 07.05.2010 DE 102010019776
(43) Veröffentlichungstag der Anmeldung: 09.11.2011
(73) Patentinhaber: Thermo Electron LED GmbH, 63505 Langenselbold (DE)
(72) Erfinder: Heeg, Hubert, 63739 Aschaffenburg (DE); Czwak, Bernd, 63486 Bruchköbel (DE); Schmidt, Bernd, 63674 Altenstadt-Heegheim (DE)
(74) Vertreter: Lang, Friedrich

(56) Entgegenhaltungen:
- DE-U1-202008 013 492
- US-A- 4 584 275
- US-A- 6 059 507

## Beschreibung

### GEBIET DER ERFINDUNG

Die Erfindung bezieht sich auf einen Inkubator mit mehreren Ein- und Ausgabestationen.

### STAND DER TECHNIK

Die Erfindung betrifft einen Inkubator als Beispiel eines Klimaschranks. Klimaschränke werden üblicherweise dazu verwendet, Objekte unter definierten klimatischen Bedingungen wie beispielsweise einer definierten Temperatur oder in einer Atmosphäre definierter Zusammensetzung aufzubewahren. Beispiele für Klimaschränke sind Wärmeschränke, Kühlschränke oder Inkubatoren, in denen Zellkulturen oder Mikroorganismen bei erhöhter Temperatur und sehr hoher Luftfeuchtigkeit und gegebenenfalls in einer mit Kohlendioxid angereicherten Atmosphäre über längere Zeit gelagert werden. Zu diesem Zweck sind die Objekte auf geeignete Objektträger aufgebracht. Objektträger sind beispielsweise die bekannten Mikrotiterplatten. Zur Aufnahme der Objektträger befindet sich im Innenraum der Klimaschränke üblicherweise mindestens eine Objektlagervorrichtung. Diese Objektlagervorrichtungen sind im Innenraum entweder feststehend oder drehbar gelagert (sogenanntes Karussell).

Der Innenraum eines Klimaschrankes ist in der Regel über eine Türe zugänglich, die groß genug ist, um eine Objektlagervorrichtung im Innenraum zu postieren bzw. zu entnehmen. Das Vorsehen einer größeren Tür erleichtert auch die Reinigung und Wartung des Innenraums eines Klimaschrankes.

Die Beschickung einer Objektlagervorrichtung mit Objektträgern erfolgt vorzugsweise nicht durch eine größere Tür wie oben erwähnt, sondern durch eine kleinere Öffnung bzw. Schleuse. Eine solche Schleuse ist in ihren Abmessungen an die durch sie einzeln hindurch zu transportierenden Objektträger angepasst. Durch die Verwendung einer kleineren Öffnung bzw. Schleuse ist es möglich, das Klima im Klimaschrank auf einem verhältnismäßig konstanten Niveau zu halten, verglichen mit der Beschickung durch eine große Haupttür.

Die Objektträger werden üblicherweise mit Hilfe einer Transportvorrichtung durch die Öffnung bzw. Schleuse in den Innenraum des Klimaschrankes hinein und auch wieder aus diesem herausgebracht. Häufig ist eine solche Transportvorrichtung in den Klimaschrank integriert und übernimmt das Be- bzw. Entladen der Objektträger durch die Schleuse sowie den Transport zwischen Schleuse und Objektlagervorrichtung. Im Allgemeinen ist gemäß des Standes der Technik jeweils eine Schleuse pro Klimaschrank vorgesehen, wobei der Transport zwischen der Schleuse und einer Objektlagervorrichtung innerhalb des Klimaschrankes wiederum mittels einer einzigen Transportvorrichtung erfolgt.

In der US 6 059 507 A wird eine Transportvorrichtung für Halbleiterwafer beschrieben. Die US 4 584 275 A beschreibt einen Durchlaufinkubator, bei dem Analysenrähmchen durch eine Inkubationskammer hindurch transportiert und dabei beispielsweise durch Bestrahlung behandelt werden. Der Inkubator besitzt eine Eingabestation, durch welche die Analysenrähmchen auf einem Träger mittels eines Schiebers in die Inkubationskammer geschoben werden. Nachdem ein Analysenrähmchen die Inkubationskammer vollständig durchlaufen hat, fällt es aus dem Träger heraus durch einen Auslass in einen Sammelbehälter. Aus der DE 20 2008 013 492 U1 ist ein anderer Klimaschrank mit mindestens zwei Ein- und Ausgabestationen bekannt. Dabei sind jeweils zwei Ein- und Ausgabestationen zu einem Paar von Stationen zusammengefasst. Dieses Paar von Ein- und Ausgabestationen ist so angeordnet bzw. konstruiert, dass eine einzelne Transportvorrichtung, die sich bevorzugt im Inneren des Klimaschrankes befindet, beide Ein- und Ausgabestationen des Paares bedienen kann. Darüber hinaus bedient dieselbe Transportvorrichtung auch die im Inneren des Klimaschrankes angeordnete Objektlagervorrichtung. Das paarige Vorsehen von Ein- und Ausgabestationen erleichtert bereits die Integration eines Klimaschrankes in ein System mit mehreren Arbeitsstationen. Dennoch erscheint es wünschenswert, eine noch einfachere Integration eines Klimaschrankes in ein System mit mehreren Arbeitsstationen zu realisieren, wobei insbesondere auch eine größere Flexibilität hinsichtlich der Systemintegration möglich sein soll.

### BESCHREIBUNG DER ERFINDUNG

Es ist die Aufgabe der vorliegenden Erfindung, einen Inkubator bereitzustellen, der sich noch besser und flexibler in ein System mit mehreren Arbeitsstationen integrieren lässt. Des Weiteren soll der erfindungsgemäße Inkubator auch eine schnellere Handhabung von Objekten erlauben.

Die Aufgabe wird gelöst durch die unabhängigen Patentansprüche. Die abhängigen Ansprüche sind auf vorteilhafte Ausführungsformen der Erfindung gerichtet.

Gemäß einem ersten Aspekt der Erfindung bezieht sich diese auf einen Inkubator gemäß Anspruch 1. Der erfindungsgemäße Inkubator weist eine Objektlagervorrichtung, eine erste Ein- und Ausgabestation zum Ein- und Ausgeben von Objekten und eine zugehörige erste Transportvorrichtung zum Positionieren von Objekten in der Objektlagervorrichtung sowie eine zweite Ein- und Ausgabestation zum Ein- und Ausgeben von Objekten und eine zugehörige zweite Transportvorrichtung zum Positionieren von Objekten in der Objektlagervorrichtung auf. Dabei interagiert die erste Transportvorrichtung nur mit der ersten Ein- und Ausgabestation als Ein- und Ausgabestation, und die zweite Transportvorrichtung interagiert nur mit der zweiten Ein- und Ausgabestation als Ein- und Ausgabestation. Die Objektlagervorrichtung dient zum Lagern von Objekten. Bei diesen Objekten kann es sich um Objekte im festen oder flüssigen Aggregatzustand handeln. Es kann sich beispielsweise um chemische Substanzen oder biologische Objekten handeln. Insbesondere kann es sich um Zellkulturen oder Mikroorganismen handeln. Diese Objekte werden in der Objektlagervorrichtung gelagert. Dabei ist es insbesondere möglich, dass die Objekte auf einem Objektträger, wie zum Beispiel einer Mikrotiterplatte, in der Objektlagervorrichtung gelagert werden. Die Objekte können jedoch auch ohne speziellen Objektträger in den Inkubator eingebracht und gelagert werden. Die Objektlagervorrichtung selbst kann unterschiedlich ausgestaltet sein. Erfindungsgemäß handelt es sich um eine drehbar gelagerte Objektlagervorrichtung, die als so genanntes Karussell bezeichnet wird. Es kann sich auch um eine Stapelvorrichtung (engl. "stacker") handeln, welche im Allgemeinen in Form eines Turmes ausgebildet ist. In einer solchen Stapelvorrichtung können verschiedene Proben bzw. Objekte in verschiedenen Fächern aufbewahrt bzw. aufgestapelt werden.

Erfindungsgemäß verfügt der Inkubator über eine erste Ein- und Ausgabestation zum Ein- und Ausgeben von Objekten und eine zugehörige erste Transportvorrichtung zum Positionieren von Objekten in der Objektlagervorrichtung. Es erfolgt somit eine Zuordnung zwischen der ersten Ein- und Ausgabestation einerseits und einer Transportvorrichtung andererseits. Die Transportvorrichtung sorgt dabei für den Transport eines eingegebenen Objektes durch die Ein- und Ausgabestation zur Objektlagervorrichtung auch für das Einbringen z.B. Hineinschieben des Objektes in die Objektlagervorrichtung. Des Weiteren dient dieselbe Transportvorrichtung dazu, die Objekte von der Objektlagervorrichtung zurück zu der Ein- und Ausgabestation zu transportieren, wobei wiederum die Transportvorrichtung auch den eigentlichen Prozess der Entnahme des Objektes aus der Objektlagervorrichtung vornimmt. Dabei kann die Transportvorrichtung ein- oder mehrteilig ausgebildet sein. Die Ein- und Ausgabestation kann unterschiedlich ausgestaltet sein. Es kann sich um eine einfache Öffnung oder aber um eine komplette Ein- und Ausgabestation mit Zubehör und/oder Schleusenfunktion handeln. Die Ein- und Ausgabestation kann dauerhaft in den Inkubator integriert sein und sich insbesondere innerhalb des Inkubators befinden. Es ist aber auch möglich, dass sich die Ein- und Ausgabestation außerhalb des Inkubators befindet und/oder lediglich modular an diesem befestigbar bzw. befestigt ist. Die zugehörige Transportvorrichtung kann konstruktiv innerhalb des Inkubators vorgesehen sein. Es ist aber auch möglich, dass die Transportvorrichtung sich außerhalb des Inkubators befindet und beispielsweise in eine modular am Inkubator vorgesehene Ein- und Ausgabestation integriert ist.

Erfindungsgemäß ist auch eine zweite Ein- und Ausgabestation zum Ein- und Ausgeben von Objekten und eine zugehörige zweite Transportvorrichtung zum Positionieren von Objekten in der Objektlagervorrichtung vorgesehen. Die erste Ein- und Ausgabestation ist nicht identisch mit der zweiten Ein- und Ausgabestation. Des Weiteren sind die erste Transportvorrichtung und die zweite Transportvorrichtung nicht identisch miteinander. Ansonsten jedoch gilt für die zweite Ein- und Ausgabestation sowie für ihre zugehörige zweite Transportvorrichtung das bezüglich der ersten Ein- und Ausgabestation und der zugehörigen ersten Transportvorrichtung Ausgesagte. Dabei kann sowohl die erste Transportvorrichtung als auch die zweite Transportvorrichtung die Objektlagervorrichtung bedienen.

Die prinzipielle konstruktive Ausgestaltung solcher Transportvorrichtungen ist im Prinzip aus dem Stand der Technik bekannt. Häufig verfügen die Transportvorrichtungen über ein bewegliches Tablett, auf dem die zu transportierenden Objekte transportiert werden. Ein Transport der Objekte kann dabei jeweils in mehrere Richtungen erfolgen: Häufig ist ein Transport in allen drei Raumdimensionen möglich. Realisiert wird dies häufig durch die Verwendung eines Liftes, der ein Anheben bzw. Absenken der zu transportierenden Objekte ermöglicht. Kombiniert wird dieses Liftsystem häufig mit einem System für horizontale Verschiebungen, welche insbesondere im Moment des Zugriffes der Transportvorrichtung auf das zu transportierende Objekt verwendet werden. Beispielsweise fährt eine Transportvorrichtung mit Tablett mit Hilfe des Tablettes unter das zu transportierende Objekt und kann dieses aufnehmen. Häufig ist für Transportzwecke auch eine Kombination einer Verschiebung in der Horizontalen mit einer Drehung um eine vertikale Achse realisiert, so dass beispielsweise das Positionieren eines Objektes in einer Objektlagervorrichtung bezogen auf den Inkubator in einer anderen Orientierung erfolgen kann als das Ein- und Ausgeben desselben Objektes.

Gemäß einer bevorzugten Ausführungsform der Erfindung ist der Inkubator so eingerichtet, dass die beiden Ein- und Ausgabestationen und ihre zugehörigen Transportvorrichtungen gleichzeitig Objekte ein- und/oder ausgeben bzw. transportieren können. Es ist also so, dass die beiden Transportvorrichtungen weitgehend unabhängig voneinander in Betrieb sein können. Beispielsweise kann gleichzeitig durch die erste Ein- und Ausgabestation ein Objekt auf die erste Transportvorrichtung eingeladen werden, während gleichzeitig ein anderes Objekt durch die zweite Transportvorrichtung an die zweite Ein- und Ausgabestation für Ausgabezwecke übergeben wird. Es ist auch denkbar, dass die erste Ein- und Ausgabestation und ihre zugehörige Transportvorrichtung mit einer Ein- oder Ausgabe eines Objektes befasst ist, während zeitgleich die zweite Transportvorrichtung ein Objekt innerhalb des Inkubators transportiert oder aber bereits in der Objektlagervorrichtung positioniert oder aber aus der Objektlagervorrichtung entnimmt. Eine solche Konfiguration des erfindungsgemäßen Inkubators ermöglicht es verglichen mit dem Stand der Technik, einen zeitlich optimierteren Ablauf von Prozessschritten zu realisieren. Die beiden Ein- und Ausgabestationen und ihre zugehörigen Transportvorrichtungen arbeiten mithin weitgehend unabhängig voneinander und jeweils für sich zeitlich optimiert.

Gemäß einer weiteren bevorzugten Ausführungsform der Erfindung ist der Inkubator so konfiguriert, dass lediglich eine der Transportvorrichtungen die Objektlagervorrichtung zur selben Zeit bedienen kann. Abgesehen von dieser Einschränkung können die erste und die zweite Transportvorrichtung jedoch wiederum unabhängig voneinander arbeiten. Die beschriebene Konfiguration ist insbesondere sinnvoll, da es sich bei der Objektlagervorrichtung um eine drehbar im Inkubator angeordnete Objektlagervorrichtung handelt. In diesem Fall wird die Objektlagervorrichtung zunächst in eine Drehposition gebracht, die einer der beiden Transportvorrichtungen den Zugriff auf ein ausgewähltes Fach bzw. eine ausgewählte Position in der Objektlagervorrichtung erlaubt. Anschließend kann die Objektlagervorrichtung in eine andere Position gedreht werden, und der Zugriff ist für die andere Transportvorrichtung nun möglich. In ähnlicher Weise kann ein Vorgang ablaufen, wenn die Objektlagervorrichtung in dem Inkubator horizontal verschiebbar angeordnet ist.

Gemäß einer bevorzugten Ausführungsform der Erfindung verfügen die Transportvorrichtungen über eine Einrichtung zum vertikalen Transport von Objekten. Dies kann beispielsweise über das bereits oben angesprochene Liftsystem realisiert werden. Dies ist insbesondere dann von Vorteil, wenn die im Inkubator zu lagernden Objekte übereinander aufgestapelt sind, beispielsweise in einer Stapelvorrichtung gelagert werden.

Gemäß einer weiteren bevorzugten Ausführungsform der Erfindung sind die beiden Transportvorrichtungen in der gleichen Weise ausgestaltet. Dies bedeutet, dass beide Transportvorrichtungen im Prinzip über die gleichen Transportmöglichkeiten hinsichtlich der Transportrichtungen und hinsichtlich der mit ihnen zu transportierenden Objekte ausgestaltet sind. Dabei müssen die Transportvorrichtungen hinsichtlich der Richtungen nicht zwangsläufig bezogen auf den Inkubator in exakt denselben Richtungen arbeiten können, stattdessen ist gemeint, dass die beiden Transportvorrichtungen über dieselbe Anzahl und Art von Bewegungsmöglichkeiten verfügen. Beispielsweise können beide Transportvorrichtungen jeweils über eine Liftfunktion, eine Drehfunktion und über eine Verschiebefunktion in der Horizontalen verfügen.

Gemäß einer alternativen Ausführungsform der Erfindung sind die beiden Transportvorrichtungen in unterschiedlicher Weise ausgestaltet. Dies kann sowohl die Richtungen des Transportes als auch die Möglichkeiten hinsichtlich der zu transportierenden Objekte betreffen. Beispielsweise kann eine der beiden Transportvorrichtungen über eine zusätzliche Verschiebefunktion verfügen, über die die andere Transportvorrichtung nicht verfügt. Es ist beispielsweise auch möglich, dass die eine Transportvorrichtung größere Objekte als die andere Transportvorrichtung transportieren kann. Die wahlweise Ausgestaltung der Transportvorrichtung in der gleichen Weise oder aber in unterschiedlicher Weise erlaubt eine bessere individuelle Anpassung des erfindungsgemäßen Inkubators an die jeweiligen Nutzerbedürfnisse und gestattet eine bessere und individuellere Systemintegration des Inkubators in eine Laborumgebung.

Gemäß einer bevorzugten Ausführungsform der Erfindung sind die beiden Ein- und Ausgabestationen des Inkubators an verschiedenen Seiten des Inkubators angeordnet. Dabei können die beiden Ein- und Ausgabestationen z.B. an benachbarten Seiten des Inkubators oder aber auch an einander gegenüberliegenden Seiten des Inkubators angeordnet sein. Es ist beispielsweise möglich, dass die erste Ein- und Ausgabestation in einer Seitenwand des Inkubators angeordnet ist, und dass sich die zweite Ein- und Ausgabestation in einer Frontfläche des Inkubators befindet. Alternativ ist es auch möglich, dass sich die erste Ein- und Ausgabestation in einer Seitenwand des Inkubators befindet, und dass sich die zweite Ein- und Ausgabestation an der Rückseite des Inkubators befindet. Es ist auch möglich, dass sich die erste Ein- und Ausgabestation an einer ersten Seitenfläche, beispielsweise links, des Inkubators befindet, und dass sich die zweite Ein- und Ausgabestation an der anderen Seitenfläche des Inkubators, beispielsweise rechts, befindet. In allen Fällen können sich die beiden Ein- und Ausgabestationen bezüglich einer Bodenfläche des Inkubators in etwa in derselben Höhe befinden. Es ist aber auch möglich, dass sich die beiden Ein- und Ausgabestationen bezogen auf eine Bodenfläche des Inkubators in unterschiedlicher Höhe befinden. Selbstverständlich ist es auch möglich, dass die beiden Ein- und Ausgabestationen nicht an verschiedenen Seiten des Inkubators, sondern an derselben Seite des Inkubators angeordnet sind. Die Ein- und Ausgabestationen können sich, bezogen auf eine Seitenfläche des Inkubators, zentral mittig an dieser oder aber seitlich versetzt zu dieser Mitte befinden.

Gemäß einer weiteren Ausführungsform der Erfindung weist der Inkubator des Weiteren eine Tür zum Entfernen der Objektlagervorrichtung auf. Diese Tür ist mithin in ihren Abmessungen deutlich größer als die beiden Ein- und Ausgabestationen zum Ein- und Ausgeben von einzelnen Objekten. Gemäß einer Ausführungsform der Erfindung kann sich eine Ein- und Ausgabestation auch innerhalb der genannten Tür befinden bzw. in diese integriert sein.

Selbstverständlich ist es auch möglich, dass zusätzlich zu der ersten und zweiten Ein- und Ausgabestation mit der jeweils zugehörigen ersten bzw. zweiten Transportvorrichtung eine dritte Ein- und Ausgabestation mit einer zugehörigen dritten Transportvorrichtung zum Positionieren von Objekten in der Objektlagervorrichtung vorgesehen ist. Dabei unterscheidet sich die dritte Transportvorrichtung wiederum sowohl von der ersten als auch von der zweiten Transportvorrichtung. In ähnlicher Weise kann auch eine vierte oder weitere Ein- und Ausgabestation samt Transportvorrichtung vorgesehen sein.

Gemäß einem weiteren Aspekt der Erfindung bezieht sich diese auf ein System mit einem Inkubator, wie er vorstehend beschrieben worden ist. Des Weiteren umfasst das erfindungsgemäße System mehrere Arbeitsstationen, die jeweils benachbart zu den Ein- und Ausgabestationen des Inkubators angeordnet sind, so dass die zu den Ein- und Ausgabestationen zugehörigen Transportvorrichtungen des Inkubators auch die Arbeitsstationen bedienen können. Beispielsweise können die Transportvorrichtungen also Objekte zu den Arbeitsstationen transportieren und in diese hinein geben sowie auch Objekte aus den Arbeitsstationen heraus entnehmen. Dabei können sich die Arbeitsstationen hinsichtlich ihrer Funktionen voneinander unterscheiden, sie können aber auch dieselbe Funktion ausüben. Als Arbeitsstationen kommen beispielsweise eine Spendervorrichtung bzw. Befüllvorrichtung (engl. "dispenser"), eine Detektionsvorrichtung (engl. "reader"), eine Waschvorrichtung (engl. "washer"), eine Schüttelvorrichtung bzw. Rührvorrichtung (engl. "shaker"), eine Versiegelungsvorrichtung (engl. "sealer") oder aber eine simple Bereitstellungsvorrichtung für Laborware bzw. zu handhabende Objekte in Frage.

Gemäß einer bevorzugten Ausführungsform der Erfindung ist der Inkubator des Systems eingerichtet, um die Arbeitsstationen zu steuern. Auf diese Art und Weise kann der erfindungsgemäße Inkubator sehr einfach mit verschiedenen Arbeitsstationen kombiniert werden, und eine Systemintegration ist deutlich erleichtert. Über den Inkubator kann somit eine Steuerung des gesamten Laborsystems, inklusive der Arbeitsstationen, erreicht werden.

Gemäß einer bevorzugten Ausführungsform verfügt der Inkubator über eine interne Steuereinheit zum Steuern des Inkubators selbst und der Arbeitsstationen. Das bedeutet, dass sich die Steuereinheit physikalisch innerhalb des Inkubators befindet. Alternativ ist es aber auch möglich, dass der Inkubator über eine externe Schnittstelle verfügt, über die der Inkubator und damit gegebenenfalls auch die Arbeitsstationen ansteuerbar sind. Beispielsweise kann über eine solche externe Schnittstelle eine Verbindung zu einem herkömmlichen Computer hergestellt werden, der auf eine Software zugreift, die ein Ansteuern des Inkubators erlaubt. Die Software kann fest auf dem Computer installiert sein, sie kann aber auch tragbar, beispielsweise in Form einer CD oder DVD oder in Form eines anderen Datenträgers bereitgestellt sein.

Die vorstehend beschriebenen Ausführungsbeispiele des Inkubators bzw. des Systems aus Inkubator und mehreren Arbeitsstationen können ganz oder teilweise miteinander kombiniert werden.

Die vorstehend beschriebene Erfindung wird noch besser verstanden werden unter Bezugnahme auf die beigefügten Zeichnungen:
- Fig. 1:: zeigt schematisch einen erfindungsgemäßen Inkubator mit zwei Ein- und Ausgabestationen sowie den jeweils zugehörigen Transportvorrichtungen; und
- Fig. 2:: zeigt schematisch eine alternative Ausführungsform eines erfindungsgemäßen Inkubators, der in ein System mit zwei Arbeitsstationen integriert ist.

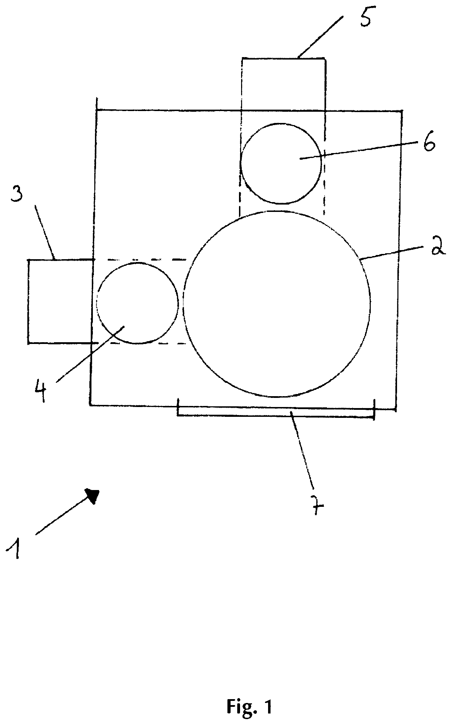

Fig. 1 zeigt einen Inkubator 1 in schematischer Darstellung. Es handelt sich dabei um eine schematische Draufsicht. Innerhalb des Inkubators befindet sich eine Objektlagervorrichtung 2, die beispielsweise in Form eines Karussells ausgebildet sein kann. Die Objektlagervorrichtung 2 verfügt über mehrere in unterschiedlichen Ebenen befindliche Fächer, es handelt sich somit um eine Stapelvorrichtung. Ein Zugriff auf die gesamte Objektlagervorrichtung 2 kann durch die große Tür 7 des Inkubators 1 erfolgen.

Im Betrieb des Inkubators 1 erfolgt eine Ein- bzw. Ausgabe von Objekten durch die beiden Ein- und Ausgabestationen 3 und 5. Die beiden Stationen 3 und 5 sind an verschiedenen Seiten des Inkubators 1 angeordnet. Im gezeigten Beispiel befinden sie sich an einander benachbarten Seiten. Im gezeigten Beispiel sind die Ein- und Ausgabestationen 3 sowie 5 in Form einer kleinen Schleuse ausgebildet. Der Ein- und Ausgabestation 3 ist dabei eine Transportvorrichtung 4 zugeordnet. Der Ein- und Ausgabestation 5 ist eine zweite Transportvorrichtung 6 zugeordnet. Die erste Transportvorrichtung 4 sorgt für das Ein- und Ausgeben von Objekten durch die Ein- und Ausgabestation 3 sowie für einen Transport zwischen der Ein- und Ausgabestation 3 einerseits und der Objektlagervorrichtung 2 andererseits. Des Weiteren ist die Transportvorrichtung 4 in der Lage, auf ihr transportierte Objekte direkt in die Objektlagervorrichtung 2 einzugeben bzw. aus dieser zu entnehmen. In analoger Art und Weise ermöglicht die Transportvorrichtung 6 ein Ein- bzw. Ausgeben von Objekten durch die Ein- und Ausgabestation 5 sowie einen Transport zwischen der Ein- und Ausgabestation 5 einerseits und der Objektlagervorrichtung 2 andererseits. Des Weiteren kann mit Hilfe der Transportvorrichtung 6 ein transportiertes Objekt direkt in die Objektlagervorrichtung 2 hineingegeben bzw. aus dieser entnommen werden. Im gezeigten Ausführungsbeispiel sind die beiden Transportvorrichtungen 4 und 6 im Wesentlichen gleichartig ausgestaltet. Sie verfügen jeweils über eine Verschiebefunktion in der Ebene, über eine Drehfunktion, sowie jeweils über eine Liftfunktion, mit deren Hilfe zu transportierende Objekte entlang einer Achse, die aus der Papierebene in Fig. 1 hinausragt, transportiert werden können. Mit Hilfe dieser Liftfunktion können Objektlagerplätze in der Objektlagervorrichtung 2, die sich in unterschiedlicher Höhe befinden, durch die Transportvorrichtungen 4 bzw. 6 erreicht werden.

Im gezeigten Ausführungsbeispiel ist der Inkubator 1 so eingerichtet, dass die beiden Ein- und Ausgabestation 3 bzw. 5 und ihre zugehörigen Transportvorrichtungen 4 bzw. 6 gleichzeitig Objekte ein- oder ausgeben bzw. transportieren können. Die beiden Einheiten aus Ein- und Ausgabestation und zugehöriger Transportvorrichtung 3 und 4 bzw. 5 und 6 arbeiten somit weitgehend unabhängig voneinander. Allerdings ist der gezeigte Inkubator des Weiteren so konfiguriert, dass lediglich eine der Transportvorrichtungen 4 oder 6 die Objektlagervorrichtung 2 zur selben Zeit bedienen kann. Die drehbar gelagerte Objektlagervorrichtung 2 wird also entweder in eine Position gebracht, die einen Zugriff der Transportvorrichtung 4 auf ein vorausgewähltes Fach ermöglicht, oder aber die Objektlagervorrichtung 2 wird in eine Position gebracht, die einen Zugriff der Transportvorrichtung 6 auf ein anderes vorausgewähltes Fach bzw. einen vorausgewählten Lagerplatz realisierbar macht. Dies ermöglicht einen sowohl Zeit sparenden als auch sicheren Prozessablauf innerhalb des Inkubators 1.

Der in Fig. 1 gezeigte Inkubator 1 kann problemlos in ein Laborsystem mit verschiedenen Arbeitsstationen integriert werden. Diese Arbeitsstationen können in der Nähe der Ein- und Ausgabestationen 3 bzw. 5 angeordnet werden (in Fig. 1 nicht dargestellt).

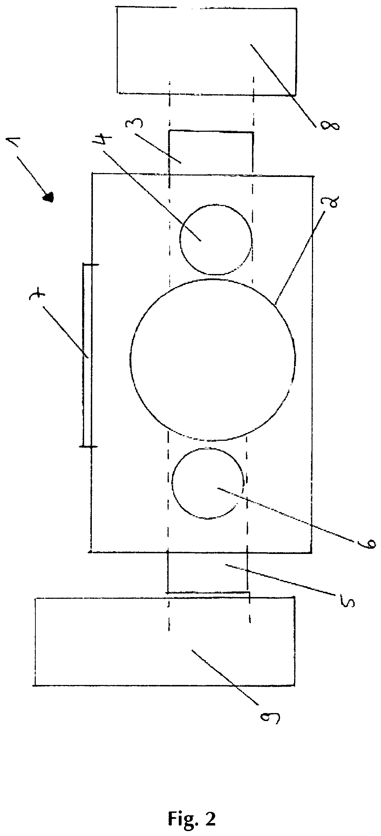

Fig. 2 zeigt in schematischer Darstellung ein System mit einem Inkubator 1 und mehreren Arbeitsstationen 8 und 9. Der Inkubator 1 verfügt wiederum über eine größere Türe 7, durch die hindurch eine Reinigung und Wartung des Inkubators 1 sowie ein Zugriff auf die komplette Objektlagervorrichtung 2 möglich ist.

Im dargestellten Ausführungsbeispiel befinden sich die beiden Ein- und Ausgabestationen 3 bzw. 5, die wiederum in Form einer Schleuse ausgestaltet sind, an einander gegenüberliegenden Seiten des Inkubators 1. Den jeweiligen Ein- und Ausgabestationen 3 bzw. 5 ist jeweils eine Transportvorrichtung 4 bzw. 6 zugeordnet. Diese Transportvorrichtungen 4 und 6 erlauben ein Ein- oder Ausgeben von Objekten durch die Ein- und Ausgabestationen 3 bzw. 5, einen Transport zwischen den Ein- und Ausgabestationen 3 bzw. 5 einerseits und der Objektlagervorrichtung 2 andererseits sowie ein Bestücken bzw. Entnehmen von Objekten in die Objektlagervorrichtung 2 hinein bzw. aus dieser heraus. Die beiden Transportvorrichtungen 4 und 6 sind wiederum in gleicher Art und Weise ausgestaltet, sie könnten aber auch in verschiedener Art und Weise ausgestaltet sein. Im gezeigten Beispiel sind beide dazu in der Lage, sowohl eine horizontale Verschiebung als auch eine Hubbewegung entlang einer aus der Papierebenen herauszeigenden Achse Z sowie eine Drehbewegung um diese Achse Z durchzuführen.

An die beiden Ein- und Ausgabestationen 3 und 5 angeschlossen sind die beiden Arbeitsstationen 8 und 9. Bei den Arbeitsstationen kann es sich um Arbeitsstationen unterschiedlicher Art handeln. Beispielsweise ist es möglich, dass es sich bei der Arbeitsstation 9 um eine Objektbereitstellungsarbeitsstation handelt, in der beispielsweise leere Objekte wie beispielsweise leere Mikrotiterplatten bereitgestellt werden, die dann durch die Ein- und Ausgabestation 5 mit Hilfe der Transportvorrichtung 6 in den Inkubator 1 hineingegeben werden. Bei der Arbeitsstation 8 kann es sich beispielsweise um eine Befüllvorrichtung handeln, in der beispielsweise ursprünglich leere Objekte mit einem bestimmten Stoff wie beispielsweise einem bestimmten Lösungsmittel oder einer organischen Probe befüllt werden. Zum Befüllen können beispielsweise leere Objekte bzw. Objektträger aus der Objektlagervorrichtung 2 mit Hilfe der Transportvorrichtung 4 entnommen werden. Sie werden mit Hilfe der Transportvorrichtung 4 zu der Ein- und Ausgabestation 3 transportiert und durch diese ausgegeben sowie der Befüllvorrichtung 8 bereitgestellt. Nach dem in der Befüllvorrichtung 8 erfolgten Befüllvorgang kann eine Rückführung der befüllten Objekte mit Hilfe der Transportvorrichtung 4 durch die Ein- und Ausgabestation 3 hindurch zurück in den Inkubator 1 und in diesem zurück in die Objektlagervorrichtung 2 erfolgen.

In dem gezeigten Beispiel ist es so, dass das System aus Inkubator 1 und Arbeitsstationen 8 und 9 durch den Inkubator 1 gesteuert wird. Dazu kann der Inkubator 1 über eine interne Steuereinheit zum Steuern des Inkubators 1 und der Arbeitsstationen 8 und 9 verfügen. Es ist aber auch möglich, dass der gezeigte Inkubator 1 über eine externe Schnittstelle verfügt, über die der gesamte Inkubator 1 zum Zwecke der Systemsteuerung ansteuerbar ist.

Mit Hilfe der Erfindung ist es also möglich, einen Inkubator auf einfache Art und Weise in eine bestehende Laborumgebung mit verschiedenen Arbeitsstationen zu integrieren. Die dort durchgeführten Prozessschritte können zeitlich optimiert und individuell ausgeführt werden.

## Patentansprüche

1. Inkubator (1), der Folgendes aufweist:
eine Objektlagervorrichtung (2), welche drehbar im Inkubator (1) angeordnet ist;
eine erste Ein- und Ausgabestation (3) zum Ein- und Ausgeben von Objekten und eine zugehörige erste Transportvorrichtung (4) zum Positionieren von Objekten in der Objektlagervorrichtung (2), wobei die erste Transportvorrichtung (4) nur mit der ersten Ein- und Ausgabestation (3) als Ein- und Ausgabestation (3) interagiert; und
eine zweite Ein- und Ausgabestation (5) zum Ein- und Ausgeben von Objekten und eine zugehörige zweite Transportvorrichtung (6) zum Positionieren von Objekten in der Objektlagervorrichtung (2), wobei die zweite Transportvorrichtung (6) nur mit der zweiten Ein- und Ausgabestation (5) als Ein- und Ausgabestation (5) interagiert.

2. Inkubator (1) gemäß Anspruch 1, wobei die beiden Ein- und Ausgabestationen (3, 5) an verschiedenen Seiten des Inkubators (1) angeordnet sind.

3. Inkubator (1) gemäß einem der vorangehenden Ansprüche, wobei die beiden Ein- und Ausgabestationen (3, 5) an benachbarten Seiten des Inkubators (1) angeordnet sind.

4. Inkubator (1) gemäß einem der Ansprüche 1 bis 2, wobei die beiden Ein- und Ausgabestationen (3, 5) an einander gegenüberliegenden Seiten des Inkubators (1) angeordnet sind.

5. Inkubator (1) gemäß einem der vorangehenden Ansprüche, wobei die Objektlagervorrichtung (2) eine Stapelvorrichtung (engl. "stacker") umfasst.

6. Inkubator (1) gemäß einem der vorangehenden Ansprüche, der so eingerichtet ist, dass die beiden Ein- und Ausgabestationen (3, 5) und ihre zugehörigen Transportvorrichtungen (4, 6) gleichzeitig Objekte ein- oder ausgeben bzw. transportieren können.

7. Inkubator (1) gemäß einem der vorangehenden Ansprüche, der so konfiguriert ist, dass lediglich eine der Transportvorrichtungen (4, 6) die Objektlagervorrichtung (2) zur selben Zeit bedienen kann.

8. Inkubator (1) gemäß einem der vorangehenden Ansprüche, wobei die Transportvorrichtungen (4, 6) über eine Einrichtung zum vertikalen Transport von Objekten verfügen.

9. Inkubator (1) gemäß einem der vorangehenden Ansprüche, wobei die beiden Transportvorrichtungen (4, 6) in der gleichen Weise ausgestaltet sind.

10. Inkubator (1) gemäß einem der Ansprüche 1 bis 9, wobei die beiden Transportvorrichtungen (4, 6) in unterschiedlicher Weise ausgestaltet sind.

11. Inkubator (1) gemäß einem der vorangehenden Ansprüche mit einer Steuereinheit zum Steuern des Inkubators (1).

12. Inkubator (1) gemäß einem der vorangehenden Ansprüche, der des Weiteren eine Tür (7) zum Entfernen der Objektlagervorrichtung (2) aufweist.

13. System, umfassend:
einen Inkubator (1) gemäß einem der Ansprüche 1 bis 12; und
mehrere Arbeitsstationen (8, 9), die jeweils benachbart zu den Ein- und Ausgabestationen (3, 5) des Inkubators (1) angeordnet sind, sodass die zu den Ein- und Ausgabestationen (3, 5) zugehörigen Transportvorrichtungen (4, 6) des Inkubators (1) die Arbeitsstationen (8, 9) bedienen können.

14. System gemäß dem vorangehenden Anspruch, wobei der Inkubator (1) eingerichtet ist, um die Arbeitsstationen (8, 9) zu steuern.

15. System gemäß dem vorangehenden Anspruch, wobei der Inkubator (1) über eine interne Steuereinheit zum Steuern des Inkubators (1) selbst und der Arbeitsstationen (8, 9) verfügt.

16. System gemäß Anspruch 14, wobei der Inkubator (1) über eine externe Schnittstelle verfügt, über die der Inkubator (1) ansteuerbar ist.

## Claims

1. Incubator (1) comprising:
an object storage device (2), which is rotatably arranged in the incubator (1);
a first input and output station (3) for inputting and outputting objects and an associated first transport device (4) for positioning objects in the object storage device (2), wherein the first transport device (4) only interacts with the first input and output station (3) as an input and output station (3); and
a second input and output station (5) for inputting and outputting objects and an associated second transport device (6) for positioning objects in the object storage device (2), wherein the second transport device (6) only interacts with the second input and output station (5) as an input and output station (5).

2. Incubator (1) according to claim 1, wherein the two input and output stations (3, 5) are arranged on different sides of the incubator (1).

3. Incubator (1) according to any of the preceding claims, wherein the two input and output stations (3, 5) are arranged on adjacent sides of the incubator (1).

4. Incubator (1) according to either claim 1 or claim 2, wherein the two input and output stations (3, 5) are arranged on opposite sides of the incubator (1).

5. Incubator (1) according to any of the preceding claims, wherein the object storage device (2) comprises a stacker.

6. Incubator (1) according to any of the preceding claims, designed such that the two input and output stations (3, 5) and the associated transport devices (4, 6) thereof can simultaneously input or output and transport objects.

7. Incubator (1) according to any of the preceding claims, configured such that only one of the transport devices (4, 6) can serve the object storage device (2) at any one time.

8. Incubator (1) according to any of the preceding claims, wherein the transport devices (4, 6) have an apparatus for transporting objects vertically.

9. Incubator (1) according to any of the preceding claims, wherein the two transport devices (4, 6) have the same design.

10. Incubator (1) according to any of claims 1 to 9, wherein the two transport devices (4, 6) have different designs.

11. Incubator (1) according to any of the preceding claims, comprising a control unit for controlling the incubator (1).

12. Incubator (1) according to any of the preceding claims, further comprising a door (7) for removing the object storage device (2).

13. System comprising:
an incubator (1) according to any of claims 1 to 12; and
a plurality of workstations (8, 9), which are each arranged adjacently to the input and output stations (3, 5) of the incubator (1) such that the transport devices (4, 6) of the incubator (1) that are associated with the input and output stations (3, 5) can serve the workstations (8, 9).

14. System according to the preceding claim, wherein the incubator (1) is designed to control the workstations (8, 9).

15. System according to the preceding claim, wherein the incubator (1) has an internal control unit for controlling the incubator (1) itself and the workstations (8, 9).

16. System according to claim 14, wherein the incubator (1) has an external interface via which the incubator (1) can be actuated.

## Revendications

1. Incubateur (1), lequel présente les éléments suivants :
un dispositif de stockage d'objets (2), lequel est disposé à rotation dans l'incubateur (1) ;
un premier poste d'entrée et de sortie (3) destiné à l'entrée et à la sortie d'objets et un premier dispositif de transport associé (4) destiné à positionner des objets dans le dispositif de stockage d'objets (2), le premier dispositif de transport (4) interagissant uniquement avec le premier poste d'entrée et de sortie (3) en tant que poste d'entrée et de sortie (3) ; et
un second poste d'entrée et de sortie (5) destiné à l'entrée et à la sortie d'objets et un second dispositif de transport associé (6) destiné à positionner des objets dans le dispositif de stockage d'objets (2), le second dispositif de transport (6) interagissant uniquement avec le second poste d'entrée et de sortie (5) en tant que poste d'entrée et de sortie (5).

2. Incubateur (1) selon la revendication 1, dans lequel les deux postes d'entrée et de sortie (3, 5) sont disposés sur des côtés différents de l'incubateur (1).

3. Incubateur (1) selon l'une des revendications précédentes, dans lequel les deux postes d'entrée et de sortie (3, 5) sont disposés sur des côtés adjacents de l'incubateur (1).

4. Incubateur (1) selon l'une des revendications 1 à 2, dans lequel les deux postes d'entrée et de sortie (3, 5) sont disposés sur des côtés opposés de l'incubateur (1).

5. Incubateur (1) selon l'une des revendications précédentes, dans lequel le dispositif de stockage d'objets (2) comprend un dispositif d'empilage.

6. Incubateur (1) selon l'une des revendications précédentes, lequel est conçu de telle sorte que les deux postes d'entrée et de sortie (3, 5) et leurs dispositifs de transport associés (4, 6) peuvent simultanément faire entrer ou faire sortir ou transporter des objets.

7. Incubateur (1) selon l'une des revendications précédentes, lequel est configuré de telle sorte qu'un seul dispositif de transport (4, 6) à la fois peut faire fonctionner le dispositif de stockage d'objets (2).

8. Incubateur (1) selon l'une des revendications précédentes, dans lequel les dispositifs de transport (4, 6) possèdent un moyen destiné au transport vertical d'objets. ,

9. Incubateur (1) selon l'une des revendications précédentes, dans lequel les deux dispositifs de transport (4, 6) sont réalisés de la même manière.

10. Incubateur (1) selon l'une des revendications 1 à 9, dans lequel les deux dispositifs de transport (4, 6) sont réalisés de manière différente.

11. Incubateur (1) selon l'une des revendications précédentes comportant une unité de commande destinée à commander l'incubateur (1).

12. Incubateur (1) selon l'une des revendications précédentes, lequel présente en outre une porte (7) destinée à retirer le dispositif de stockage d'objets (2).

13. Système comprenant:
un incubateur (1) selon l'une des revendications 1 à 12 ; et
une pluralité de postes de travail (8, 9), chacun étant disposé de façon adjacente aux postes d'entrée et de sortie (3, 5) de l'incubateur (1), de sorte que les dispositifs de transport (4, 6) de l'incubateur (1) associés aux postes d'entrée et de sortie (3, 5) peuvent faire fonctionner les postes de travail (8, 9).

14. Système selon la revendication précédente, dans lequel l'incubateur (1) est conçu pour commander les postes de travail (8, 9).

15. Système selon la revendication précédente, dans lequel l'incubateur (1) possède une unité de commande interne destinée à commander l'incubateur (1) lui-même et les postes de travail (8, 9).

16. Système selon la revendication 14, dans lequel l'incubateur (1) possède une interface externe par l'intermédiaire de laquelle l'incubateur (1) peut être commandé.
